# EUROPEAN PATENT SPECIFICATION

(11) **EP 3 773 526 B1**
(45) Date of publication and mention of the grant of the patent: **17.07.2024**
(21) Application number: 19722208.6
(22) Date of filing: 08.04.2019
(51) Int. Cl.: A61K 31/015, A61K 31/045, A61K 31/164, A61K 31/232, A61K 31/385, A61K 31/593, A61P 29/02, A61P 25/02

(54) **COMPOSITION FOR THE TREATMENT OF PERIPHERAL NEUROPATHIES**
ZUSAMMENSETZUNG ZUR BEHANDLUNG PERIPHERER NEUROPATHIEN
COMPOSITION POUR LE TRAITEMENT DE NEUROPATHIES PÉRIPHÉRIQUES

(30) Priority: 10.04.2018 IT 201800004355
(43) Date of publication of application: 17.02.2021
(73) Proprietor: Sochim International SpA, 20010 Cornaredo (MI) (IT)
(72) Inventor: EIGENMANN, Carlo Vittorio, 20010 Cornaredo (MI) (IT)
(74) Representative: Villa, Livia
(86) International application number: PCT/IB2019/052872
(87) International publication number: WO 2019/197967

(56) References cited:
- EP-A1- 2 921 167
- EP-A1- 3 130 336
- WO-A1-2016/146453
- HESSELINK J M ET AL: "Therapeutic utility of palmitoylethanolamide in the treatment of neuropathic pain associated with various pathological conditions: a case series", JOURNAL OF PAIN RESEARCH, DOVE MEDICAL PRESS, GB, vol. 5, 1 January 2012 (2012-01-01), pages 437 - 442, XP002757426, ISSN: 1178-7090, [retrieved on 20121026], DOI: 10.2147/JPR.S32143
- EUN YEONG LIM ET AL: "Food-Derived Natural Compounds for Pain Relief in Neuropathic Pain", BIOMED RESEARCH INTERNATIONAL, vol. 2016, 1 January 2016 (2016-01-01), pages 1 - 12, XP055450350, ISSN: 2314-6133, DOI: 10.1155/2016/7917528
- FINE PERRY G ET AL: "Cannabinoids for Neuropathic Pain", CURRENT PAIN AND HEADACHE REPORTS, CURRENT SCIENCE, US, vol. 18, no. 10, 28 August 2014 (2014-08-28), pages 1 - 9, XP035397930, ISSN: 1531-3433, [retrieved on 20140828], DOI: 10.1007/S11916-014-0451-2

## Description

### FIELD OF THE INVENTION

The present invention relates to a composition for the treatment of peripheral neuropathy, in particular of the pain associated therewith, as well as mixed pain.

### BACKGROUND ART

Peripheral neuropathy is a functional disorder affecting the peripheral nervous system. The nervous system consists of two components: the central nervous system, constituted of the encephalon and the spinal cord, and the peripheral nervous system, constituted of nerves which connect the central nervous system to the muscles, skin and internal organs. The peripheral nervous system is the part which, when damaged, determines neuropathy. Neuropathy may concern one single nerve (mononeuropathy), multiple nerves (multiple mononeuropathy) or assume a symmetrical and bilateral distribution (polyneuropathy). Mononeuropathy is generally a consequence of a traumatic injury, local compression (with "crushing" of the nerve) or inflammatory or ischemic processes. The symptomatology is therefore localised and limited to the area of innervation of the damaged nerve.

Polyneuropathy, meanwhile, is determined principally by deficiency, dysmetabolic, toxic, infective or iatrogenic factors.

The symptoms of peripheral neuropathy may assume a notable variety of forms and intensities. In general, they are divided into three groups: motor symptoms, sensory symptoms and vegetative symptoms.

The motor symptoms which may occur may be extremely varied: they range from the sensation of clumsiness in multiple fine motor movements of the fingers to the reduction of strength in the legs, to the sensation of fatigue when performing movements such as walking or climbing the stairs. Motor problems may be linked to one or multiple limbs; in the event that the difficulty concerns the lower limbs, for example, problems with standing or walking can be experienced.

The signs and symptoms of a sensory nature type are extremely varied; generally, they onset slowly, may be sporadic in nature and are often initially underestimated. Less frequently, they have a continuous nature and it is unlikely that they maintain the same intensity throughout the day. Sensory symptoms may manifest in the form of varyingly intense pain, burning, tingling, torpor and so on.

The neurological signs are extremely varied; indeed, they may include, for example alteration of reflexes, variously noticeable lack of strength, increase, reduction or absence of responses to sensory stimuli (respectively: hyperaesthesia, hypoaesthesia or anaesthesia), allodynia (anomalous response to stimuli which should not normally cause pain) and hyperalgesia (excessive response to slightly painful stimuli).

From an etiopathogenetic point of view, the pain may be classified as: nociceptive (direct activation of nociceptive receptors), neuropathic (injury to or dysfunction of the central and/or peripheral nervous system), mixed (with the presence of all the previous components) and psychogenic (activated by psycho-relational factors).

Neuropathic pain, a direct consequence of an injury or dysfunction of the nervous system, is defined as either central or peripheral depending on whether the problem concerns the central nervous system (CNS) or the peripheral one (PNS) or both. The mechanism underlying the onset of neuropathic pain is not yet totally clear, but it seems to depend on the fact that the damaged nerves become hyperexcitable, thus causing an alteration of the mechanism which, through the nerve fibres, transmits the stimuli felt from the periphery to the brain structures responsible for the perception of pain. It happens, therefore, that the severity of the pain often does not non correspond to the severity of the underlying condition. For example, postherpetic neuralgia may occur with intense pain even without any cutaneous manifestation (rash) or other sign of residual infection by herpes zoster. The onset of neuropathic pain is often slow, sometimes preceded by paraesthesia and dysaesthesia which may evolve into pain, while in other cases, the onset is abrupt.

As regards analgesic therapies, neuropathic pain (unlike other types of pain) is scarcely controlled by traditional analgesics.

Compared with nociceptive pain, neuropathic pain responds less well to monotherapy and the administration of multiple drugs is often required. At present, the first-line drugs for the treatment of neuropathic and/or mixed pain are tricyclic antidepressants, serotonin-noradrenaline reuptake inhibitors (SNRI), antiepileptic drugs, and opioids. These drugs offer, however, limited clinical benefits and expose patients to the risk of undesired effects. Alternative treatments are provided using endocannabinoids. For example, Hesselink J.M. et al. (Journal of Pain Research, 2012, vol. 5, pages 437-442) discloses the therapeutic utility of palmitoylethanolamide, also used in combination with *R*-alpha-lipoic acid and vitamin D3, for the treatment of neuropathic pain. The European Patent application EP 3 130 336 A1 (2017) discloses nutraceutical compositions comprising palmitoylethanolamide and β-caryophyllene for the treatment of allodynia.

The object of the present invention is therefore to provide a new therapy proposal without undesired effects for the treatment of peripheral neuropathy, in particular of the pain associated therewith, as well as mixed pain.

### SUMMARY OF THE INVENTION

Said object has been achieved by a composition comprising at least one endocannabinoid being palmitoylethanolamide, at least one phytoterpene being β-caryophyllene, and at least one component selected from mixtures of alpha-lipoic acid and vitamin D, as stated in Claim 1.

In a further aspect, the present invention concerns said composition for use in the treatment of the peripheral neuropathy, in particular of the pain associated therewith, as well as mixed pain.

As will be clear in the detailed description and the working examples provided for illustrative, non-limiting purposes, the composition according to the invention has surprisingly allowed to boost the anti-inflammatory action and therefore to improve the analgesic action on neuropathic and mixed pain.

The characteristics and advantages of the present invention will become clearer in the following detailed description and embodiments provided by way of illustrative, non-limiting examples. The present invention is defined by the claims. Any subject-matter falling outside the scope of the claims is provided for information only.

### DETAILED DESCRIPTION OF THE INVENTION

The invention therefore relates to a composition comprising at least one endocannabinoid being palmitoylethanolamide, at least one phytoterpene being β-caryophyllene, and at least one component selected from mixtures of alpha-lipoic acid and vitamin D.

For the purposes of the present disclosure, the term "endocannabinoid" encompasses anandamide (or arachidonoylethanolamide, AEA), docosatetraenoylethanolamide (DEA), homo-linolenylethanolamide (HEA), 2-arachidonoylglycerol (2-AG), 2-arachidonoyl-glyceril-ether (noladin ether, 2-AGE), virodamine, N-arachidonoyl dopamine (NADA), palmitoylethanolamide (PEA), oleoylethanolamide (OEA), and mixtures thereof.

The endocannabinoids have a lipid nature and derive from a polyunsaturated fatty acid, i.e. arachidonic acid. They are produced from phospholipid biosynthetic precursors and activate type 1 cannabinoid receptors (CB1), which are extremely abundant in the brain but also in peripheral tissues, and type 2 cannabinoid receptors (CB2), which are expressed, meanwhile, principally in immune system cells, but are also expressed by the glial cells affecting the CNS. In the central nervous system, the endocannabinoids perform a neuromodulatory function, very often of a retrograde kind. In this way, they play an important role in various kinds of synaptic plasticity and in the cognitive, motor, sensory, and affective processes correlated thereto. Furthermore, in certain pathological conditions, whether acute or chronic, of the CNS, such as during epilepsy or in neuroinflammatory and neurodegenerative illnesses, by activating both CB1 and CB2 receptors, the endocannabinoids can perform a pro-homeostatic and neuroprotective role.

According to the present invention, the at least one endocannabinoid comprised in the claimed composition is palmitoylethanolamide (PEA). PEA has, as its principal mechanism of action, an increase in the expression of CB2 receptors through a genomic mechanism involving the activation of PPAR-α. The stimulation of the CB2 receptors, expressed above all in the microglia, promotes the release of endogenous opioids, with an analgesic effect, and the release of anti-inflammatory cytokines.

For the purposes of the present disclosure, the term "phytoterpene" encompasses a terpene of plant origin, preferably selected from limonene, myrcene, α-pinene, linalool, β-caryophyllene, caryophyllene oxide, nerolidol, phytol, alpha-humulene, isocaryophyllene, and mixtures thereof.

According to the present invention, the at least one phytoterpene comprised in the claimed composition is β-caryophyllene.

β-caryophyllene is a natural bicyclic sesquiterpene present in many essential oils and extracts from a wide variety of plants, including cannabis sativa, hemp, black cumin, cloves, hops, basil, oregano, black pepper, lavender, rosemary, cinnamon, ylang-ylang, and copaiba. Therefore, for the purposes of the present invention, β-caryophyllene may be provided as such or in the form of an essential oil or in the form of a fluid extract or a dry extract/powder of one or multiple of the plants listed above. In preferred embodiments, β-caryophyllene is in the form of a dry extract of black pepper. β-caryophyllene binds selectively with the CB2 receptors, acting as an agonist. As mentioned above, the stimulation of the CB2 receptors, expressed above all in the microglia, promotes the release of endogenous opioids, with an analgesic effect, and the release of anti-inflammatory cytokines.

Preferably, in the composition according to the invention, said at least one endocannabinoid is present in an amount higher than said at least one phytoterpene.

In preferred embodiments of the invention, said at least one endocannabinoid and said at least one phytoterpene are in a weight ratio of 2:1 to 200:1. Preferably, said at least one endocannabinoid and said at least one phytoterpene are in a weight ratio of 2:1 to 150:1, more preferably 20:1 to 100:1.

In preferred embodiments, said at least one endocannabinoid and said at least one phytoterpene are in a weight ratio of 35:1 to 45:1.

As may be observed from the above weight ratios, a minimal amount of said at least one phytoterpene with respect to the amount of at the least one endocannabinoid is sufficient to obtain a synergic effect and an improvement in the treatment of neuropathic pain and mixed pain. Without wishing to be bound to any theory, it is believed that the synergic action is a result of the combined action on the CB2 receptors. The endocannabinoid (specifically PEA) acts by increasing expression of CB2 receptors and phytoterpene (specifically β-caryophyllene) acts by binding as an agonist, with such CB2 receptors performing a greater analgesic and anti-inflammatory action than that ascribable to the individual components.

In certain embodiments, the composition according to the invention comprises up to 50 wt% of said at least one endocannabinoid, more preferably up to 30 wt%.

For the purposes of the present invention, unless otherwise specified, the term "wt%" means % of weight based on the weight the composition according to the invention.

In other embodiments, the composition according to the invention comprises up to 5 wt% of at least one phytoterpene, more preferably up to 1 wt%.

The composition according to the invention also comprises at least one component selected from mixtures of alpha-lipoic acid and vitamin D.

In certain embodiments, the composition according to the invention comprises up to 0.05 wt% of vitamin D, more preferably up to 0.001 wt%.

The term "vitamin D" means one group of liposoluble prohormones constituted of vitamin D1, vitamin D2, vitamin D3, vitamin D4, vitamin D5, or a mixture thereof.

Preferably, the vitamin D is vitamin D3. Indeed, studies have shown vitamin D3, or cholecalciferol, to be the most effective form of vitamin D to ensure the right concentrations of this nutrient in the blood. Vitamin D supplementation is advisable since:
- vitamin D has been proved to have an anti-inflammatory effect, by down-regulating expression of various pro-inflammatory cytokines,
- recent studies have shown that vitamin D reduces microglia activation, which is a process which supports neuroinflammation,
- it performs a neuroprotective action by stimulating the release of anti-inflammatory cytokines (IL-10, IL-4 and TGF-β), which are characteristic of the expression of M2-type microglia phenotype (cytoprotective),
- it promotes the synthesis of neurotrophic factors NGF and BDNF which determine neuronal survival, differentiation, and function.

In other embodiments, the composition according to the invention comprises up to 40 wt% of lipoic acid, more preferably up to 35 wt%.

Lipoic acid supplementation is advisable in the treatment of neuropathic pain, since it is a molecule with a strong antioxidising power originating from the chemical/physical properties thereof as an amphoteric molecule and of a redox pair. The involvement of the oxidation processes in the onset and worsening of neuropathic pain has been demonstrated in numerous studies which have highlighted the neuroprotective role thereof in painful neuropathic diseases. It has furthermore been demonstrated that lipoic acid has neurotrophic functions and induces increased synthesis of NGF (Nerve Growth Factor) which reduces lipid peroxidation of the nerve tissue and the formation of free radicals. Lipoic acid is furthermore a fundamental cofactor in the Krebs cycle, assuming a fundamental role in the energy production processes in cells, and in particular neuronal energy production, and stimulates the production of glutathione, whose deficiency causes neuronal cell damage.

Numerous clinical studies have demonstrated the effectiveness of lipoic acid in improving nerve conduction parameters in patients with peripheral neuropathy, with increased function and improvement of painful and sensory symptomatology.

In preferred embodiments, the composition according to the invention is in the form of a unit dose.

Preferably, said unit dose comprises 1-3000 mg of said at least one endocannabinoid, more preferably 50-2000 mg.

In particularly preferred embodiments, said unit dose comprises 100-1500 mg of said at least one endocannabinoid.

Preferably, said unit dose comprises up to 300 mg of said at least one phytoterpene, more preferably 1-200 mg.

In particularly preferred embodiments, said unit dose comprises 1-100 mg of said at least one phytoterpene.

Preferably, said unit dose further comprises up to 2000 UI of vitamin D, more preferably 1-1500 UI.

Preferably, said unit dose further comprises 1-3000 mg of lipoic acid, more preferably 50-1500 mg.

In preferred embodiments, said unit dose comprises 1-3000 mg of said at least one endocannabinoid, up to 300 mg of said at least one phytoterpene, up to the 2000 UI of vitamin D, and 1-3000 mg of lipoic acid.

In particularly preferred embodiments, said unit dose comprises 50-2000 mg of said at least one endocannabinoid, 1-200 mg of said at least one phytoterpene, 1-1500 UI of vitamin D, and 50-1500 mg of lipoic acid.

Even more preferable are the unit doses comprising 50-2000 mg palmitoylethanolamide, 1-200 mg β-caryophyllene, 1-1500 UI vitamin D, and 50-1500 mg lipoic acid.

Even more preferable are the compositions, as well as the unit doses, comprising:

| | |
|---|---|
| - palmitoylethanolamide | 600 mg |
| - β-caryophyllene | 15 mg |
| - vitamin D3 | 1000 UI (25 µg) |
| - lipoic acid | 800 mg |
| or | |
| - palmitoylethanolamide | 300 mg |
| - β-caryophyllene | 7.5 mg |
| - vitamin D3 | 500 UI (12.5 µg) |
| - lipoic acid | 400 mg |
| or | |
| - palmitoylethanolamide | 1200 mg |
| - β-caryophyllene | 15 mg |
| - vitamin D3 | 1000 UI (25 µg) |
| - lipoic acid | 800 mg |
| or | |
| - palmitoylethanolamide | 600 mg |
| - β-caryophyllene | 15 mg |
| - vitamin D3 | 2000 UI (50 µg) |
| - lipoic acid | 800 mg |
| or | |
| - palmitoylethanolamide | 1200 mg |
| - β-caryophyllene | 15 mg |
| - vitamin D3 | 2000 UI (50 µg) |
| - lipoic acid | 800 mg |
| or | |
| - palmitoylethanolamide | 600 mg |
| - β-caryophyllene | 15 mg |
| - vitamin D3 | 2000 UI (50 µg) |
| - lipoic acid | 1000 mg |
| or | |
| - palmitoylethanolamide | 600 mg |
| - β-caryophyllene | 25 mg |
| - vitamin D3 | 1000 UI (25 µg) |
| - lipoic acid | 800 mg |
| or | |
| - palmitoylethanolamide | 1200 mg |
| - β-caryophyllene | 25 mg |
| - vitamin D3 | 2000 UI (50 µg) |
| - lipoic acid | 800 mg |

It should be understood that the aspects specified as preferable for the individual components should be deemed to be likewise preferable in the unit dose embodiments disclosed above.

The composition according to the invention, also in the form of a unit dose, may further comprise pharmaceutically acceptable excipients. The term "excipient" means a compound or a mixture thereof suitable for use in a formulation for the treatment of peripheral neuropathy, in particular of the pain associated therewith. For example, an excipient for use in a pharmaceutical formulation generally must not cause an adverse reaction in a patient, nor must it significantly inhibit the effectiveness of the composition. Suitable excipients include: acidifiers, acidity regulators, anti-caking agents, antioxidants, bulking agents, firming agents, gelling agents, coating agents, modified starches, sequestrants, thickeners, sweeteners, diluents, disintegrants, glidants, colourings, binders, lubricants, stabilisers, adsorbents, preservatives, humectants, flavourings, filmogenic substances, emulsifiers, wetting agents, release retardants and mixtures thereof.

Preferably, said excipients are potassium sorbate, sodium benzoate, ε-polylysine, sucralose, maltodextrin, citric acid, sodium carbonate, calcium carbonate, magnesium carbonate, magnesium stearate, stearic acid, polyethylene glycol, natural starch, partially hydrolysed starch, modified starch, lactose, calcium phosphate, calcium carbonate, calcium sulphate, polyvinylpyrrolidone, silica, colloidal silica, precipitated silica, magnesium silicates, aluminium silicates, sodium lauryl sulphate, magnesium lauryl sulphate, methacrylate copolymers, sodium dehydroacetate, xanthan gum, guar gum, tara gum, locust bean gum, fenugreek gum, gum arabic, alginic acid, sodium alginate, propylene glycol alginate, sodium croscarmellose, polyvinylpolypyrrolidone, glyceryl behenate, titanium dioxide, indigo carmine, cellulose, modified cellulose, calcium carboxymethylcellulose, sodium carboxymethylcellulose, microcrystalline cellulose, ethylcellulose, gelatine, ethylcellulose, hydroxyethylcellulose, hydroxypropylcellulose, polydextrose, carrageenan, methylcellulose, saccharose, saccharose esters, sorbitol, xylitol, dextrose, fructose, maltitol, tragacanth gum, pectin, agar-agar, carboxypolymethylene, hydroxypropylmethylcellulose, tragacanth, mannitol, or a mixture thereof.

In certain embodiments, the composition according to the invention consists essentially of at least one endocannabinoid, at least one phytoterpene, and at least one component selected from alpha-lipoic acid, vitamin D and mixtures thereof. The expression "consists essentially of" means that at least one endocannabinoid, at least one phytoterpene, and at least one component chosen from alpha-lipoic acid, vitamin D, and mixtures thereof are the sole ingredients present in the composition to be active in the treatment of the inflammation and of the neuropathic pain, while any further components or excipients do not interfere with the action thereof. It should be understood that all the aspects specified above as preferable and advantageous for the composition and the components thereof should be deemed to be likewise preferable and advantageous also for these embodiments. In other embodiments, the composition according to the invention consists of at least one endocannabinoid, at least one phytoterpene, at least one component selected from alpha-lipoic acid, vitamin D, and mixtures thereof, and optionally pharmaceutically acceptable excipients. It should be understood that all the aspects specified above as preferable and advantageous for the composition and the components thereof should be deemed to be likewise preferable and advantageous also for these embodiments.

The composition of the present invention may be prepared by using methods known to a person skilled in the art. Indeed, for oral administration, the components may, for example, be mixed as such or with one or multiple excipients, sealed in soft-gel capsules or in a solid form, such as a tablet, mini-tablet, micro-tablet, granule, micro-granule, pellet, multiparticulate, or micronised particulate, or powder or in the form of a solution, emulsion, gel, vial, drops, or spray.

Preferably, the composition according to the invention is in the form of a tablet.

In a further aspect, the present invention concerns the composition disclosed above for use in the treatment of peripheral neuropathy, in particular of the pain associated therewith. As already stated, the composition according to the invention has surprisingly allowed to improve the analgesic action on neuropathic and mixed pain.

The composition according to the invention may be administered orally, buccally, or sublingually.

Preferably, the composition according to the invention is administered orally.

More preferably, the composition according to the invention is administered at least once a day. For the purposes of the present invention, the term "day" means a time period of 24±4 hours.

In preferred embodiments, the composition according to the invention is administered once or twice a day in the form of a unit dose, as disclosed above.

It should be understood that all the possible combinations of the preferred aspects of the components of the composition, as stated above, have been disclosed and therefore are likewise deemed preferable.

It should furthermore be understood that all the aspects specified as preferable and advantageous for the composition and the components thereof should be deemed to be likewise preferable and advantageous for the preparation and the uses of said composition.

Below are working examples of the present invention provided for illustrative purposes.

### EXAMPLES

### Example 1.

The following composition was prepared:

| | |
|---|---|
| - palmitoylethanolamide | 600 mg |
| - β-caryophyllene | 15 mg |
| - vitamin D3 | 1000 UI (25 µg) |
| - lipoic acid | 800 mg |

### Example 2.

The following composition was prepared:

| | |
|---|---|
| - palmitoylethanolamide | 300 mg |
| - β-caryophyllene | 7.5 mg |
| - vitamin D3 | 500 UI (12.5 µg) |
| - lipoic acid | 400 mg |

### Example 3. (Reference Example)

The following composition was prepared:

| | |
|---|---|
| - oleoylethanolamide | 300 mg |
| - oxide caryophyllene | 7.5 mg |
| - vitamin D3 | 500 UI (12.5 µg) |
| - lipoic acid | 400 mg |

**Example 4.** (Reference Example)

The following composition was prepared:

| | |
|---|---|
| - 2-arachidonoylglycerol | 1000 mg |
| - alpha-humulene | 7 mg |
| - vitamin D3 | 750 UI (18.75 µg) |
| - lipoic acid | 500 mg |

### Example 5. (Reference Example)

The following composition was prepared:

| | |
|---|---|
| - anandamide | 400 mg |
| - nerolidol | 80 mg |
| - vitamin D3 | 1000 UI (25 µg) |
| - lipoic acid | 1000 mg |

### Example 6. (Reference Example)

The following composition was prepared:

| | |
|---|---|
| - palmitoylethanolamide | 600 mg |
| - β-caryophyllene | 15 mg |

### Example 7.

| | |
|---|---|
| - palmitoylethanolamide | 1200 mg |
| - β-caryophyllene | 15 mg |
| - vitamin D3 | 1000 UI (25 µg) |
| - lipoic acid | 800 mg |

### Example 8.

| | |
|---|---|
| - palmitoylethanolamide | 600 mg |
| - β-caryophyllene | 15 mg |
| - vitamin D3 | 2000 UI (50 µg) |
| - lipoic acid | 800 mg |

### Example 9.

| | |
|---|---|
| - palmitoylethanolamide | 1200 mg |
| - β-caryophyllene | 15 mg |
| - vitamin D3 | 2000 UI (50 µg) |
| - lipoic acid | 800 mg |

### Example 10.

| | |
|---|---|
| - palmitoylethanolamide | 600 mg |
| - β-caryophyllene | 15 mg |
| - vitamin D3 | 2000 UI (50 µg) |
| - lipoic acid | 1000 mg |

### Example 11.

| | |
|---|---|
| - palmitoylethanolamide | 600 mg |
| - β-caryophyllene | 25 mg |
| - vitamin D3 | 1000 UI (25 µg) |
| - lipoic acid | 800 mg |

### Example 12.

| | |
|---|---|
| - palmitoylethanolamide | 1200 mg |
| - β-caryophyllene | 25 mg |
| - vitamin D3 | 2000 UI (50 µg) |
| - lipoic acid | 800 mg |

## Claims

1. A composition comprising at least one endocannabinoid being palmitoylethanolamide, at least one phytoterpene being β-caryophyllene, and at least one component selected from mixtures of alpha-lipoic acid and vitamin D.

2. The composition of claims 1, wherein said at least one endocannabinoid is present in an amount higher than said at least one phytoterpene.

3. The composition of claim 2, wherein said at least one endocannabinoid and said at least one phytoterpene are in a weight ratio of 2:1 to 200:1.

4. The composition of claim 3, wherein said at least one endocannabinoid and said at least one phytoterpene are in a weight ratio of 2:1 to 150:1.

5. The composition of claim 3, wherein said at least one endocannabinoid and said at least one phytoterpene are in a weight ratio of 20:1 to 100:1.

6. The composition of any one of the claims 1-5, comprising up to 50 wt% of said at least one endocannabinoid, preferably up to 30 wt%, based on the weight of the composition.

7. The composition of any one of claims 1-6, comprising up to 5 wt% of at least one phytoterpene, preferably up to 1 wt%, based on the weight of the composition.

8. The composition of any one of claims 1-7, comprising vitamin D up to 0.05 wt%, preferably up to 0.001 wt%, based on the weight of the composition.

9. The composition of any one of claims 1-8, comprising lipoic acid up to 40 wt%, preferably up to 35 wt%, based on the weight of the composition.

10. The composition of any one of claims 1-9, in the form of a unit dose comprising 1-3,000 mg of said at least one endocannabinoid, up to 300 mg of said at least one phytoterpene, up to 2000 UI of vitamin D, and 1-3000 mg of lipoic acid.

11. The composition of claim 10, in the form of a unit dose comprising 50-2000 mg of said at least one endocannabinoid, 1-200 mg of said at least one phytoterpene, 1-1500 UI of vitamin D, and 50-1500 mg of lipoic acid.

12. The composition of claim 11, in the form of a unit dose comprising:
| | |
|---|---|
| - palmitoylethanolamide | 600 mg |
| - β-caryophyllene | 15 mg |
| - vitamin D3 | 1000 IU (25 µg) |
| - lipoic acid | 800 mg |

13. The composition of claim 11, in the form of a unit dose comprising:
| | |
|---|---|
| - palmitoylethanolamide | 300 mg |
| - β-caryophyllene | 7.5 mg |
| - vitamin D3 | 500 IU (12.5 µg) |
| - lipoic acid | 400 mg |

14. The composition of any one of claims 1-13 for use in the treatment of peripheral neuropathies, in particular of pain ascribable thereto, and mixed pain.

## Patentansprüche

1. Zusammensetzung, umfassend zumindest ein Endocannabinoid, das Palmitoylethanolamid ist, zumindest ein Phytoterpen, das β-Caryophyllen ist, und zumindest eine Komponente ausgewählt aus Mischungen von Alpha-Liponsäure und Vitamin D.

2. Zusammensetzung nach Anspruch 1, wobei das zumindest eine Endocannabinoid in einer Menge vorhanden ist, die höher als das zumindest eine Phytoterpen ist.

3. Zusammensetzung nach Anspruch 2, wobei das zumindest eine Endocannabinoid und das zumindest eine Phytoterpen in einem Gewichtsverhältnis von 2:1 bis 200:1 sind.

4. Zusammensetzung nach Anspruch 3, wobei das zumindest eine Endocannabinoid und das zumindest eine Phytoterpen in einem Gewichtsverhältnis von 2:1 bis 150:1 sind.

5. Zusammensetzung nach Anspruch 3, wobei das zumindest eine Endocannabinoid und das zumindest eine Phytoterpen in einem Gewichtsverhältnis von 20:1 bis 100:1 sind.

6. Zusammensetzung nach einem der Ansprüche 1-5, umfassend bis zu 50 Gew.-% des zumindest einen Endocannabinoids, bevorzugt bis zu 30 Gew.-%, basierend auf dem Gewicht der Zusammensetzung.

7. Zusammensetzung nach einem der Ansprüche 1-6, umfassend bis zu 5 Gew.-% von zumindest einem Phytoterpen, bevorzugt bis zu 1 Gew.-%, basierend auf dem Gewicht der Zusammensetzung.

8. Zusammensetzung nach einem der Ansprüche 1-7, umfassend Vitamin D bis zu 0,05 Gew.-%, bevorzugt bis zu 0,001 Gew.-%, basierend auf dem Gewicht der Zusammensetzung.

9. Zusammensetzung nach einem der Ansprüche 1-8, umfassend Liponsäure bis zu 40 Gew.-%, bevorzugt bis zu 35 Gew.-%, basierend auf dem Gewicht der Zusammensetzung.

10. Zusammensetzung nach einem der Ansprüche 1-9 in der Form einer Einheitsdosis, umfassend 1-3.000 mg des zumindest einen Endocannabinoids, bis zu 300 mg des zumindest einen Phytoterpens, bis zu 2.000 UI Vitamin D und 1-3.000 mg Liponsäure.

11. Zusammensetzung nach Anspruch 10 in der Form einer Einheitsdosis, umfassend 50-2000 mg des zumindest einen Endocannabinoids, 1-200 mg des zumindest einen Phytoterpens, 1-1500 UI Vitamin D und 50-1500 mg Liponsäure.

12. Zusammensetzung nach Anspruch 11 in der Form einer Einheitsdosis, umfassend:
- Palmitoylethanolamid 600 mg
- β-Caryophyllen 15 mg
- Vitamin D3 1000 IU (25 µg)
- Liponsäure 800 mg.

13. Zusammensetzung nach Anspruch 11 in der Form einer Einheitsdosis, umfassend:
- Palmitoylethanolamid 300 mg
- β-Caryophyllen 7,5 mg
- Vitamin D3 500 IU (12,5 µg)
- Liponsäure 400 mg.

14. Zusammensetzung nach einem der Ansprüche 1-13 zur Verwendung bei der Behandlung peripherer Neuropathien, insbesondere von Schmerzen, die darauf zurückzuführen sind, und gemischten Schmerzen.

## Revendications

1. Composition comprenant au moins un endocannabinoïde qui est le palmitoyléthanolamide, au moins un phytoterpène qui est le β-caryophyllène, et au moins un composant sélectionné dans des mélanges d'acide alpha-lipoïque et de vitamine D.

2. Composition selon la revendication 1, dans laquelle le ou lesdits endocannabinoïdes sont présents dans une quantité supérieure à celle du ou desdits phytoterpènes.

3. Composition selon la revendication 2, dans laquelle le ou lesdits endocannabinoïdes et le ou lesdits phytoterpènes ont un rapport de poids de 2: 1 à 200:1.

4. Composition selon la revendication 3, dans laquelle le ou lesdits endocannabinoïdes et le ou lesdits phytoterpènes ont un rapport de poids de 2: 1 à 150:1.

5. Composition selon la revendication 3, dans laquelle le ou lesdits endocannabinoïdes et le ou lesdits phytoterpènes ont un rapport de poids de 20: 1 à 100: 1.

6. Composition selon l'une quelconque des revendications 1-5, comprenant jusqu'à 50 % en poids du ou desdits endocannabinoïdes, de préférence jusqu'à 30 % en poids, sur la base du poids de la composition.

7. Composition selon l'une quelconque des revendications 1-6, comprenant jusqu'à 5 % en poids d'au moins un phytoterpène, de préférence jusqu'à 1 % en poids, sur la base du poids de la composition.

8. Composition selon l'une quelconque des revendications 1-7, comprenant de la vitamine D jusqu'à 0,05 % en poids, de préférence jusqu'à 0,001 % en poids, sur la base du poids de la composition.

9. Composition selon l'une quelconque des revendications 1-8, comprenant de l'acide lipoïque jusqu'à 40 % en poids, de préférence jusqu'à 35 % en poids, sur la base du poids de la composition.

10. Composition selon l'une quelconque des revendications 1-9, sous la forme d'une dose unitaire comprenant 1-3000 mg du ou desdits endocannabinoïdes, jusqu'à 300 mg du ou desdits phytoterpènes, jusqu'à 2000 UI de vitamine D, et 1-3000 mg d'acide lipoïque.

11. Composition selon la revendication 10, sous la forme d'une dose unitaire comprenant 50-2000 mg du ou desdits endocannabinoïdes, 1-200 mg du ou desdits phytoterpènes, 1-1500 UI de vitamine D, et 50-1500 mg d'acide lipoïque.

12. Composition selon la revendication 11, sous la forme d'une dose unitaire comprenant :
| | |
|---|---|
| - palmitoyléthanolamide | 600 mg |
| - β-caryophyllène | 15 mg |
| - vitamine D3 | 1000 IU (25 µg) |
| - acide lipoïque | 800 mg. |

13. Composition selon la revendication 11, sous la forme d'une dose unitaire comprenant :
| | |
|---|---|
| - palmitoyléthanolamide | 300 mg |
| - β-caryophyllène | 7,5 mg |
| - vitamine D3 | 500 IU (12,5 µg) |
| - acide lipoïque | 400 mg. |

14. Composition selon l'une quelconque des revendications 1-13, destinée à être utilisée dans le traitement de neuropathies périphériques, en particulier de la douleur qui en découle, et d'une douleur mixte.
